# EUROPEAN PATENT APPLICATION

(11) **EP 2 549 249 A1**
(43) Date of publication of application: **23.01.2013**
(21) Application number: 11174895.0
(22) Date of filing: 21.07.2011
(51) Int. Cl.: G01J 5/00, G01J 5/02, G01J 5/08

(54) **Non-contact type temperature sensing device with constant distance measurement and temperature measuring method thereof**

(71) Applicant: Rossmax International Ltd., Taipei 114 (TW)
(72) Inventor: Chi, Yu-Chiao, 114 Taipei (TW); Chen, Chiung-Hsien, 114 Taipei (TW)
(74) Representative: TER MEER - STEINMEISTER & PARTNER GbR

(57) **Abstract**

A non-contact type temperature sensing device (10) includes an emitting unit (110), a detecting unit (120) and a single chip microprocessor (11). The emitting unit (110) emits a signal to a temperature-measured body. The detecting unit (120) receiving a reflected signal emitted by the emitting unit (110) and reflected by the temperature-measured body. The single chip microprocessor (11) is electrically connected to the emitting unit (110) and the detecting unit (120). The single chip microprocessor (11) determines a measuring distance between non-contact type temperature sensing device (11) and the temperature-measured body according to the reflected signal. When the measuring distance is located within a preset measuring range, the single chip microprocessor (11) outputs an enabling signal of measuring temperature.

## Description

### BACKGROUND OF THE INVENTION

### Field of the Invention

The present invention relates to a non-contact type temperature sensing device, and in particular to a non-contact type temperature sensing device with constant distance measurement.

### Description of Prior Art

Temperature is a physiological indicator of life body and can inform the health state of the life body. There are some commercially-available contact type thermometers for measuring forehead or skin temperature, however, human body under measurement may has risks of infected vectors during temperature measuring by the contact type thermometer. Therefore, non-contact type thermometer is disclosed to prevent the problem mentioned above.

The non-contact type thermometer has an infrared light sensor for sensing the intensity of the infrared radiation and converting the sensed intensity to a proportional voltage signal, and then showing it on the display via suitable mathematical calculations. However, the variation of voltage signal is in reverse proportional to a measuring distance between the non-contact type thermometer and the temperature-measured body P, namely, when measuring temperature of a temperature-measured body at different measuring distance, the measuring result are also different. The temperature-measured body is closer to the sensor, the measuring result is higher. In order to effectively prevent the measuring distance from affecting the precision of the thermometer sensor, the thermometer must design with a designation of measuring distance.

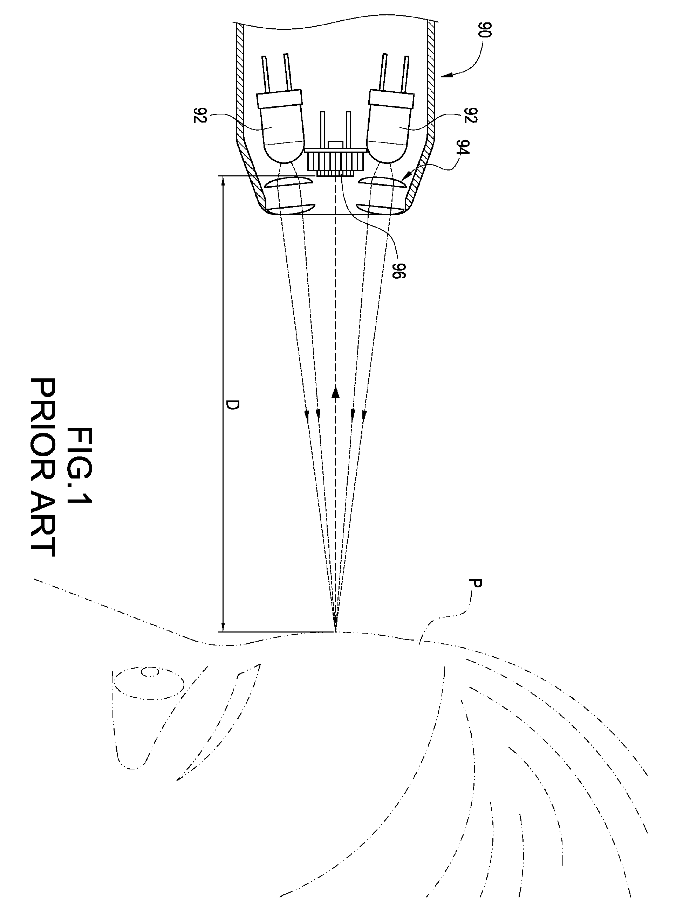

Reference is made to Fig. 1, which is a scheme view of a conventional infrared thermometer comprising optical aiming system. The infrared thermometer comprising optical aiming system 90 includes two light sources 92, two light convergent units 94 and a thermometer 96. Each light source 92 emits light to a temperature-measured body P via each light convergent unit 94. When the distance between the thermometer 96 and the temperature-measured body P is exactly equal to a preset distance D, the two light emitted by the light sources converges to a single spot so as to reduce measuring deviations due to different measuring distances.

However, the infrared thermometer comprising optical aiming system has disadvantages, such as temperature-measured body P cannot view the lights and the spot, so that users cannot measure temperature by themselves.

### SUMMARY OF THE INVENTION

Accordingly, the present invention provides a non-contact type temperature sensing device with constant distance measurement.

Moreover, the present invention provides a non-contact type temperature measuring method.

Therefore, the present invention provides a non-contact type temperature sensing device with constant distance measurement, the non-contact type temperature sensing device includes an emitting unit, a detecting unit and a single chip microprocessor. The emitting unit emits a signal to a temperature-measured body. The detecting unit receives a reflected signal emitted by the emitting unit and reflected by the temperature-measured body. The single chip microprocessor is electrically connected to the emitting unit and the detecting unit and determines a measuring distance between the non-contact type temperature sensing device and the temperature-measured body according to the reflected signal. When the measuring distance is located within a preset measuring range, the single chip microprocessor outputs an enabling signal of measuring temperature.

The present invention further provides a non-contact type temperature measuring method, comprising: at first, using an emitting unit emitting a signal to a temperature-measured body and then using a detecting unit receiving a reflected signal reflected by the temperature-measured body. Finally, using a single chip processor determining whether the reflected signal is located within a preset measuring range and if the reflected signal is located within a preset measuring range, the single chip processor outputs an enabling signal of measuring temperature.

The non-contact type temperature sensing device of the present invention detecting the measures distance between the non-contact type temperature sensing device and the temperature-measured body in advance, and when the measuring distance is located within the preset measuring range, the temperature sensing device prompts the user to measure body temperature or directly measuring body temperature to prevent the erroneous measurement and the temperature of the temperature-measured body can be automatically measured.

### BRIEF DESCRIPTION OF DRAWING

The features of the invention believed to be novel are set forth with particularity in the appended claims. The invention itself, however, may be best understood by reference to the following detailed description of the invention, which describes an exemplary embodiment of the invention, taken in conjunction with the accompanying drawings, in which:
Fig. 1 is a scheme view of a conventional infrared thermometer comprising optical aiming system;
Fig. 2 is a scheme view of a non-contact type temperature sensing device with constant distance measurement according to the present invention;
Fig. 3 is a circuit block diagram of the non-contact type temperature sensing device;
Fig. 4 is another circuit block diagram of the non-contact type temperature sensing device;
Fig. 5 is a flow chart illustrating a method for measuring temperature of the non-contact type temperature sensing device.

### DETAILED DESCRIPTION OF THE INVENTION

A preferred embodiment of the present invention will be described with reference to the drawings.

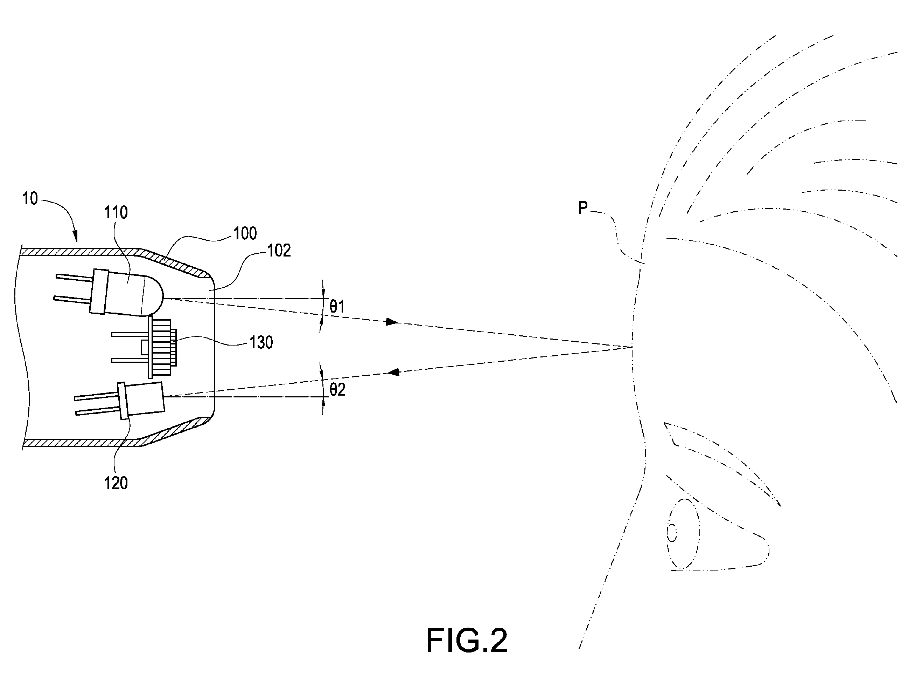

Referenced is made to Fig. 2, which is a scheme view of a non-contact type temperature sensing device with constant distance measurement according to the present invention. The non-contact type temperature sensing device 10 detects a measuring distance between a temperature-measured body P and the non-contact type temperature sensing device 10 in advance and then senses at least a body temperature of the temperature-measured body P when the measuring distance is located within a preset measuring range, so as to prevent erroneous measurement caused by different measuring distances between the temperature-measured body P and the non-contact type temperature sensing device 10 and to provide precision measuring results.

The non-contact type temperature sensing device 10 includes a housing 100, an emitting unit 110, a detecting unit 120 and a temperature sensing unit 130. The housing 100 has a window 102. The emitting unit 110, the detecting unit 120 and the temperature sensing unit 130 are disposed within the housing 100 and adjacent to the window 102. The emitting unit 110 is tilted with respect to the detecting unit 120 and has first angle θ1 with the normal of the window 102; and the detecting unit 120 is tilted with respect to the emitting unit 110 and has a second angle θ2 with the normal of the window 102 so that the signal emitted from the emitting unit 110 and reflected by the temperature-measured body P can be detected by the detecting unit 120.

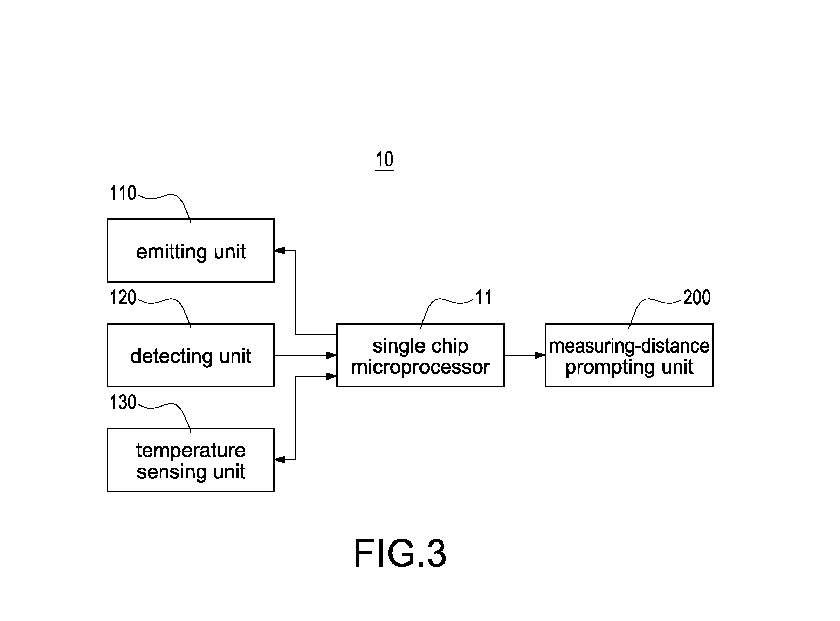

Referenced is made to Fig. 3, which is a circuit block diagram of the non-contact type temperature sensing device according to the present invention. The non-contact type temperature sensing device includes a single chip microprocessor 11, the emitting unit 110, the detecting unit 120, the temperature sensing unit 130 and a measuring-distance identification unit 200.

Referring also to Fig. 2, the emitting unit 110 emits a signal to the temperature-measured body P. The emitting unit 100 may be an invisible light emitting unit, a visible light emitting unit or an ultrasonic wave emitting unit. The invisible light emitting unit emits ultraviolet light signals (wavelength located between 200 and 400 nm) or infrared light signals (wavelength located between 700 and 14000nm). The visible light emitting unit emitting visible light signals have wavelength of 400 to 700nm. In this embodiment, the emitting unit 110 is an infrared light emitting diode and emits infrared light signal to the temperature-measured body P with the first angle θ1.

The detecting unit 120 receives a reflected signal emitted by the emitting unit 110 and reflected by the temperature-measured body P. The detecting unit 120 may be an invisible light detecting unit, a visible light detecting unit or an ultrasonic wave detecting unit and use corresponding to the invisible light emitting unit, the visible light emitting unit and the ultrasonic wave emitting unit, respectively. The invisible light detecting unit detects ultraviolet light signals (wavelength located between 200 and 400 nm) or infrared light signals (wavelength located between 700 and 14000nm). The visible light detecting unit detects visible light signals have wavelength of 400 to 700nm. In this embodiment, the detecting unit 120 is, but not limited to, an infrared phototransistor, other equivalent elements can be used without departing from the scope of the present invention.

The single chip microprocessor 11 receives the reflected signal and determines a measuring distance between the non-contact type temperature sensing device 10 and the temperature-measured body P, and outputs a constant distance identification signal or an enabling signal of measuring temperature when the measuring distance is located within a preset measuring range. The constant distance identification signal is delivered to the measuring-distance identification unit 200 and the enabling signal of measuring temperature is delivered to the temperature sensing unit 130.

In the practical application, the single chip processor 11 may be, but not limited to, an application specific integrated circuit (ASIC) or a field programmable gate arrays (FPGA), other equivalent elements can be used without departing from the scope of the present invention.

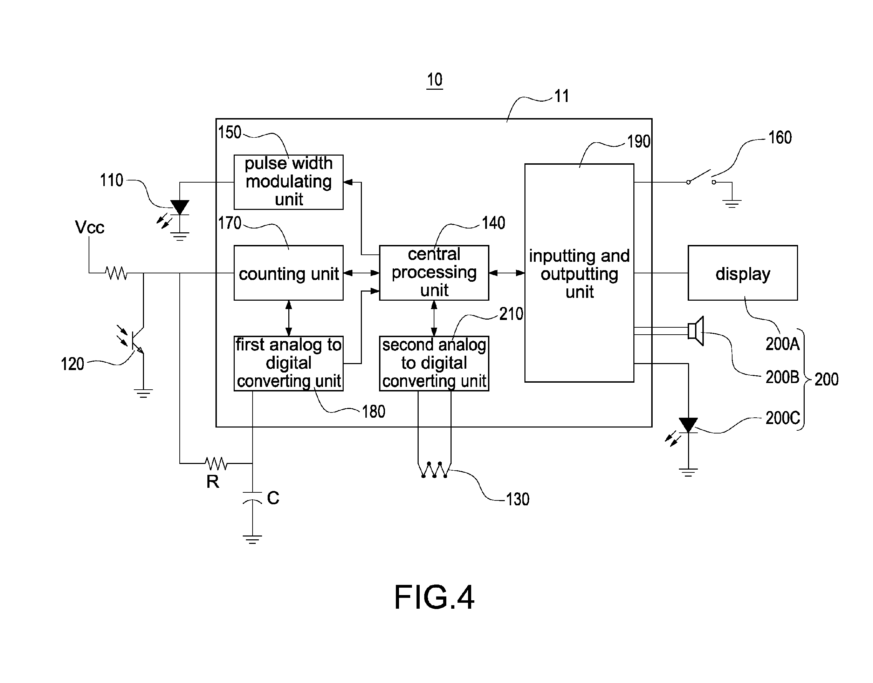

Reference is made to Fig. 4, which is another circuit block diagram of the non-contact type temperature sensing device according to the present invention. The single chip processor 11 includes a central processing unit 140, a pulse width modulating (PWM) unit 150, a counting unit 170, a first analog to digital converting (ADC) unit 180, an inputting and outputting unit 190 and a second analog to digital converting (ADC) unit 210. The non-contact type temperature sensing device 10 further includes a switching unit 160. The central processing unit 140 is electrically connected to the PWM unit 150, the counting unit 170, the first ADC unit 180, the inputting and outputting unit 190 and the second ADC unit 210. The task of the central processing unit 140 is to coordinate and direct all the data transmission and operations between the units.

The PWM unit 150 is electrically connected to the emitting unit 110 and the central processing unit 140. The PWM unit 150 has a pulse width modulating signal (PWM signal). The PWM signal controls the working duty cycle of the emitting unit 110, namely, the turn-on and the turn-off time of the emitting unit 110, so that the emitting unit 110 emits a pulse signal with the working duty cycle. Moreover, the central processing unit 140 simultaneously controls the emitting times of the pulse signal.

The switching unit 160 is disposed on the housing 100 (shown in Fig. 2) and electrically connected to the inputting and outputting unit190. User can activate or inactive the non-contact type temperature sensing device 10 by pressing the switching unit 160. And user can press the switch unit 160 to start the emitting unit 110 to emit pulse signal and to start the temperature sensing unit 130 to measure temperature of the temperature-measured body P.

The counting unit 170 is electrically connected to the detecting unit 120 and the central processing unit 140. The counting unit 170 receives the reflected signal and determines whether the pulse signal emitted by the emitting unit 110 and the reflected signal have the same working duty cycle and the emitting times is the same as so as to prevent erroneous measurement caused by environment noise (for example, noise caused by sunlight, light generated by fluorescent tube or infrared light emitted by remove control).

The first ADC unit 180 is electrically connected to the detecting unit 120 and the central processing unit 140. The reflected signal is converted to a reflected signal with analog form by a filtering circuit composed of a resistor R and a capacitor C and is delivered to the first ADC unit 180. The first ADC unit 180 receives the reflected signal with analog form and converts the reflected signal with analog form to a reflected signal with digital form and transmits the signal to the central processing unit 140.

The central processing unit 140 has a preset measuring range. The central processing unit 140 intelligently determines the reflected signal is located within the preset measuring range or not and then judges the measuring distance between the non-contact type temperature sensing device 10 and the temperature-measured body P. The intelligently determination is determined by the conditions, such as skin tone, skin gloss, skin fineness of the temperature-measured body P and then judges the measuring distance of the non-contact type temperature sensing device 10 and the temperature-measured body P.

If the central processing unit 140 judges the reflected signal with digital form is located within the preset measuring range, the central processing unit 140 determines that the measuring distance between the non-contact type temperature sensing device 10 and the temperature-measured body P is located at a suitable distance and outputs a constant distance identification signal corresponding to the intensity of the reflected signal. The constant distance identification signal is delivered to the measuring-distance identification unit 200 via the inputting and outputting unit 190 to turn on the measuring-distance identification unit 200. The measuring-distance identification unit 200 outputs a constant distance identification information so as to prompt the user using the non-contact type temperature sensing device 10 that the measuring distance is located within the preset measuring range and can measure the body temperature of the temperature-measured body P. The measuring-distance identification unit 200 may be a display 200A, a speaker 200B or an illuminant element 200C. The measuring-distance identification unit 200 can bring different audio frequencies, lighting colors or icons according to the constant distance prompting signal. The display 200A may be a liquid crystal display (LCD), the speaker 200B may be a buzzer , the illuminant element 200C may be a light emitting diode. However, those examples can be variously changed or modified within the scope of the present invention.

Moreover, the central processing unit 140 judges the distance between the non-contact type temperature sensing device 10 and the temperature-measured body P and outputs an enabling signal of measuring temperature to activate the temperature sensing unit 130 to measuring the body temperature of the temperature-measured body P. In this embodiment, the temperature sensing unit 130 is an infrared temperature sensor, and preferably a thermal resistor used to sense infrared radiation generated by the temperature-measured body P and then transmit a body temperature to the second ADC unit 210.

The second ADC unit 210 receives the body temperature and converts the body temperature to a correspondingly temperature signal and transmits the signal to the central processing unit 140.

In the practical application, the temperature sensing unit 130 senses the body temperature of the temperature-measured body P in a preset measuring range and accesses at least a body temperature. The body temperature is delivered to the second ADC unit 210 and the second ADC unit 210 converts the body temperature with analog form to at least a temperature signal with digital form. The temperature is delivered to the central processing unit 140.

The detecting way of the temperature sensing unit 130 is as follows. The non-contact type temperature sensing device 10 is moved forward and backward to accesses at least a body temperature. The body temperature is delivered to the second ADC unit 210 and converted to a temperature signal and delivered to the central processing unit 140. When the non-contact type temperature sensing device 10 is moved within the preset measuring range, the temperature signal is gradually increased. When the non-contact type temperature sensing device 10 is over the preset measuring range, the temperature signal is reduced. The central processing unit 140 accesses the largest temperature signal and delivers the temperature signal with environment temperature compensation to the display 200A via the inputting and outputting unit 190 and the display 200A to display it.

Moreover, the central processing unit 140 simultaneously judges the temperature signal is larger than a preset temperature range or not. If the temperature signal is larger than the preset temperature range, the central processing unit 140 ends all operations of the non-contact type temperature sensing device 10 to prevent the non-contact type temperature sensing device 10 from damage caused by sensing over-high temperatures. Furthermore, the central processing unit 140 also judges the temperature signal is smaller than the preset temperature range or not, and if the temperature signal is smaller than the preset temperature range, the display 200A is activated to display "Lo" signal.

In conclusions, the measuring step of the non-contact type temperature sensing device 10 is summarized as following:
First, the emitting unit 110 emits a signal to a temperature-measured body P, and then the detecting unit 120 receives a reflected signal emitted by the emitting unit 110 and reflected by the temperature-measured body P.
Finally, the single chip processor 11 determines that the reflected signal is located within the preset measuring range or not, and if the result is yes, the single chip processor 11 generates a constant distance identification signal or an enabling signal of measuring temperature.

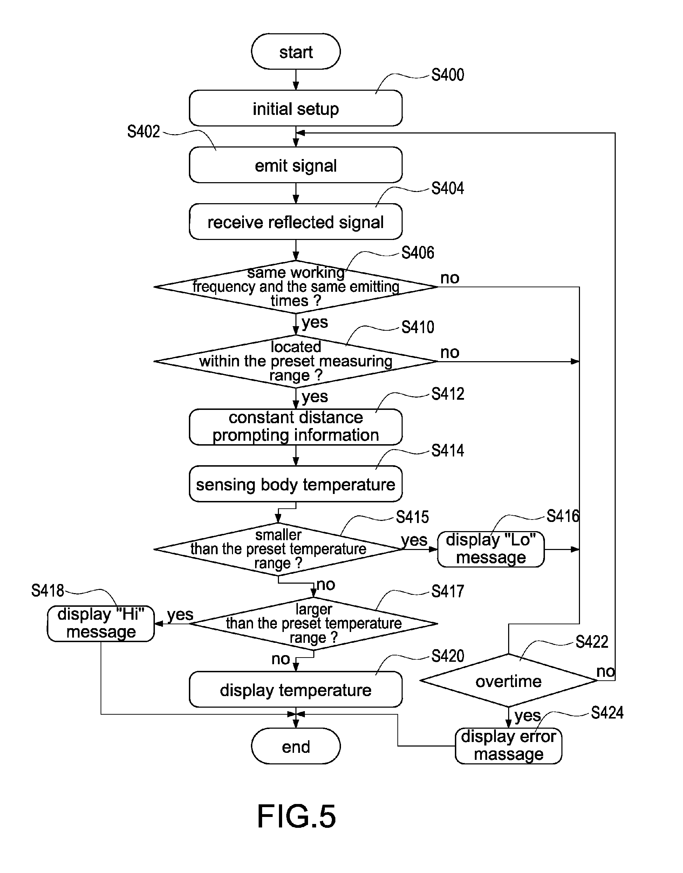

Reference is made to Fig. 5, which is a flow chart illustrating a method for measuring temperature with the non-contact type temperature sensing device. When using the non-contact type temperature sensing device10, users must activate the non-contact type temperature sensing device 10 by the switching unit 160. When the non-contact type temperature sensing device 10 is activated, the control processing unit 140 returns the non-contact type temperature sensing device 10 to initial setup (step S400).

After that, the emitting unit 110 emits a pulse signal corresponding to the emitting times (step S402), namely, the pulse signal has the working duty cycle. The pulse signal transmits to the temperature-measured body P and reflected by the temperature-measured body P. The pulse signal is controlled by the PWM signal generated by the PWM unit 150 and controls the working duty cycle of the emitting unit 110.

The detecting unit 120 receives the pulse reflected signal reflected by the temperature-measured body P (step S404) and delivers the pulse reflected signal to the counting unit 170 and the first ADC unit 180.

The counting unit 170 determines whether the pulse signal and the pulse reflected signal have the same working duty cycle and the same emitting times (step S406). If the counting unit 170 determines that the pulse signal and the pulse reflected signal do not have the same working duty cycle and the same emitting times, the central processing unit 140 is used to determine whether the non-contact type temperature sensing device 10 is overtime or not (step S422).

If the central processing unit 140 determines the non-contact type temperature sensing device 10 is overtime, the central processing unit 140 activates the display 200A to output an error massage (step S424).

After step S422, if the central processing unit 140 determines the non-contact type temperature sensing device 10 is not overtime, the central processing unit 140 returns the procedure to the step S402 and uses the emitting unit 110 to emit pulse signal.

After step S406, if the counting unit 170 determined that the pulse reflected signal and the pulse signal have the same working duty cycle and emitting times, the counting unit 170 delivers the pulse reflected signal to the central processing unit 140.

The central processing unit 140 has a preset measuring range. The central processing unit 140 judges a measuring distance between the non-contact type temperature sensing device 10 and the temperature-measured body P by determines that the pulse reflected signal is located within the preset measuring range or not (step S410).

If the central processing unit 140 determined that the pulse reflected signal is not located within the preset measuring range, and central processing unit 140 further determines that the non-contact type temperature sensing device 10 is detecting overtime or not (step S422).

After step S422, if the central processing unit 140 determines the non-contact type temperature sensing device 10 is overtime, the central processing unit 140 activates the display 200A to output an error massage (step S424).

After step S422, if the central processing unit 140 determines the non-contact type temperature sensing device 10 is not overtime, the central processing unit 140 returns the procedure to the step S402 and uses the emitting unit 110 to emit pulse signal.

After step S410, if the central processing unit 140 judges that the pulse reflected signal is located within the preset measuring range, the central processing unit 140 transmits a constant distance identification signal, which is corresponding to the reflected signal, to the measuring-distance identification unit 200 and activates the measuring-distance identification unit 200 to produce a constant distance prompting information (step S412) so as to prompt user that the non-contact type temperature sensing device 10 can measure the body temperature of the temperature-measured body P. The measuring-distance prompting unit 200 may be a display 200A, a speaker 200B or an illuminant element 200C and bring different audio frequencies, lighting colors or icons according to the constant distance identification signal, respectively.

The central processing unit 140 delivers an enabling signal of measuring temperature to the temperature sensing unit 130 and activates the temperature sensing unit 130 to sense a body temperature of the temperature-measured body P (step S414). The temperature sensing unit 130 accesses at least a body temperature with analog form and delivers the analog signal to the second ADC unit 210. The second ADC unit 210 converts the body temperature to at least a temperature signal with digital form and delivers the temperature signal to the central processing unit 140.

The central processing unit 140 has a preset temperature range. The central processing unit 140 judges whether the temperature signal is smaller than the preset temperature range (step S415). If the central processing unit 140 judges the temperature signal is smaller than the preset temperature range, the central processing unit 140 activates the display 200A to display "Lo" message (step S416), and then central processing unit 140 further determines that the non-contact type temperature sensing device 10 is overtime or not (step S422).

If the central processing unit 140 determines that the non-contact type temperature sensing device 10 is overtime, the central processing unit 140 activates the display 200A to output an error massage (step S424).

After step S422, if the central processing unit 140 determines that the non-contact type temperature sensing device 10 is not overtime, the central processing unit 140 returns the procedure to the step S402 and uses the emitting unit 110 to emit pulse signal.

After step S415. If the central processing unit 140 judges that the temperature signal is not smaller than the preset temperature range, the central processing unit 140 further judges that the temperature signal is larger than the preset temperature range or not (step S417).

If the central processing unit 140 judges that the temperature signal is not larger than the preset temperature range, the central processing unit 140 transmits the temperature signal to the display 200A via environment temperature compensation.

After step S417, if the central processing unit 140 judges that the temperature signal is larger than the preset temperature range, the display 200A displays "Hi" message and the central processing unit 140 ends the operation of the non-contact type temperature sensing device 10 to prevent the non-contact type temperature sensing device 10 from damage caused by detecting over-high temperatures.

In conclusion, the non-contact type temperature sensing device 10 of the present invention detecting the measures distance between the non-contact type temperature sensing device 10 and the temperature-measured body P in advance, and when the measuring distance is located within the preset measuring range, the temperature sensing device 10 prompts the user to measure body temperature or directly measuring body temperature to prevent the erroneous measurement and the temperature of the temperature-measured body P can be automatically measured.

## Claims

1. A non-contact type temperature sensing device with constant distance measurement (10), comprising:
an emitting unit (110) emitting a signal to a temperature-measured body;
a detecting unit (120) receiving a reflected signal emitted by the emitting unit (110) and reflected by the temperature-measured body; and
a single chip microprocessor (11) electrically connected to the emitting unit (110) and the detecting unit (120);
wherein the single chip microprocessor (11) determines a measuring distance between the non-contact type temperature sensing device (10) and the temperature-measured body according to the amplitude of the reflected signal; when the measuring distance is located within a preset measuring range, the single chip microprocessor (11) outputs an enabling signal of measuring temperature.

2. The non-contact type temperature sensing device (10) in claim 1, wherein the emitting unit (110) is a visible light emitting unit and the detecting unit (120) is a visible light detecting unit.

3. The non-contact type temperature sensing device (10) in claim 1, wherein the emitting unit (110) is an invisible light emitting unit and the detecting unit (120) is an invisible light detecting unit.

4. The non-contact type temperature sensing device (10) in claim 1, wherein the emitting unit (110) is an ultrasonic wave emitting unit and the detecting unit (120) is an ultrasonic wave detecting unit.

5. The non-contact type temperature sensing device (10) according to any one of the preceding claims, wherein the single chip microprocessor (11) comprising:
a central processing unit (140);
a first analog to digital converting (ADC) unit (180) electrically connected to the detecting unit (120) and the central processing unit (140);
a second analog to digital converting (ADC) unit (210) electrically connected the central processing unit (140); and
an inputting and outputting unit (190) electrically connected to the central processing unit (140).

6. The non-contact type temperature sensing device (10) as claimed in any one of the preceding claims, further comprising:
a temperature sensing unit (130) electrically connected to the second ADC unit (210), the temperature sensing unit (130) receiving the enabling signal of measuring temperature and sensing at least a body temperature from the temperature-measured body; and
a measuring-distance identification unit (200) electrically connected to the inputting and outputting unit (190) and providing a constant distance identification information.

7. The non-contact type temperature sensing device (10) as claimed in any one of the preceding claims, wherein the single chip microprocessor (11) further comprising a pulse width modulating (PWM) unit (150) electrically connected to the emitting unit (110) and the central processing unit (140) for controlling working duty cycle and emitting times of the emitting unit (110) so that the emitting unit (110) emits a pulse signal with pulse width modulation.

8. The non-contact type temperature sensing device (10) as claimed in any one of the preceding claims, wherein the single chip microprocessor (11) further comprising a counting unit (170) electrically connected to the detecting unit (120).

9. The non-contact type temperature sensing device (10) in any one of claim 6, 7 or 8, wherein the measuring-distance identification unit (200) is a display (200A), a speaker (200B) or a luminous element (200C).

10. The non-contact type temperature sensing device (10) in claim 9, wherein the display (200A) is adapted to show the body temperature sensed by the temperature sensing unit.

11. A non-contact type temperature measuring method, comprising:
a. using an emitting unit (110) emitting a signal to a temperature-measured body;
b. using a detecting unit (120) receiving a the above emitting signal reflected by the temperature-measured body;
c. using a single chip processor (11) determining whether the reflected signal is located within a preset measuring range, where the temperature sensing device may start detecting the temperature of the temperature-measured body;
d. the single chip processor (11) will output an enabling signal for measuring temperature whenever the above reflected signal is located within a preset measuring range.

12. The non-contact type temperature measuring method in claim 11, further comprising a following step before the step a :
using the single chip processor (11) to provide a pulse width modulating (PWM) unit (150), the PWM unit (150) generating a PWM signal and conducting the PWM signal to the emitting unit (110) to control a working duty cycle of the emitting unit (110).

13. The non-contact type temperature measuring method in claim 11 or 12, further comprising a following step after the step b :
using the single chip processor (11) to provide a counting unit (170), the counting unit (170) compares whether the signal emitted by the emitting unit (110) and the reflected signal have the same working duty cycle.

14. The non-contact type temperature measuring method as claimed in any one of the preceding claims 11-13, further comprising a step e after the step d:
e. using a temperature sensing unit (130) to access at least a body temperature.

15. The non-contact type temperature measuring method as claimed in any one of the preceding claims 11-14, further comprising a step f after the step e:
f. using the single chip processor (11) to judge whether the temperature reading is located within the preset temperature range.
